# EUROPEAN PATENT SPECIFICATION

(11) **EP 1 609 786 B1**
(45) Date of publication and mention of the grant of the patent: **03.08.2011**
(21) Application number: 04291616.3
(22) Date of filing: 25.06.2004
(51) Int. Cl.: C07D 313/08

(54) **5-alkenone-3,3-dimethyl-benzoxepines derivatives useful for the preparation of antidiabetic pentadienoic acids**
5-Alkenon-3,3-dimethyl-benzoxepinderivate und ihre Verwendung in der Herstellung von antidiabetischen Pentadiensäuren
Dérivés de 5-alcénone-3,3-diméthyl-benzoxépine utiles dans la préparation d'acides pentadiènoiques antidiabétiques

(43) Date of publication of application: 28.12.2005
(73) Proprietor: Merck Sante, 69008 Lyon (FR)
(72) Inventor: Brunet, Michel, 69780 Toussieu (FR); Le Borgne, Guy, 45300 Pithiviers le Vieil (FR)
(74) Representative: Neyret, Daniel Jean Marie

(56) References cited:
- WO-A-00/39113
- EVANS D A ET AL: "Asymmetric synthesis of the benzoquinoid ansamycin antitumor antibiotics: total synthesis of (+)-macbecin" JOURNAL OF ORGANIC CHEMISTRY, vol. 15, no. 2, 15 January 1993 (1993-01-15), pages 471-485, XP002304336
- ARCADI A ET AL: "The reaction of aryl iodides with hindered alpha,beta,gamma,delta-dienones in the presence of the [Pd(OAc)2(PPh3)2]-trialkylammonium formate reagent" JOURNAL OF ORGANOMETALLIC CHEMISTRY, vol. 312, no. 2, 23 September 1986 (1986-09-23), pages C27-C32, XP002304337
- AARESKJOLD K ET AL: "Total synthesis of 3,4,3',4'-tetradehydro-beta,beta-carotene- 2,2'-dione" ACTA CHEMICA SCANDINAVICA B, vol. 38, no. 1, 1984, pages 43-47, XP002304338

## Description

### FIELD OF THE INVENTION

The present invention relates to 5-alkenone-3,3-dimethyl-benzoxepine derivatives and their use for the preparation of pentadienoic derivatives.

### BACKGROUND OF THE INVENTION

5-(3,3-dimethyl-2,3-dihydrobenzoxepin-5-yl)-2,4-pentadienoic acid derivatives (formula II): 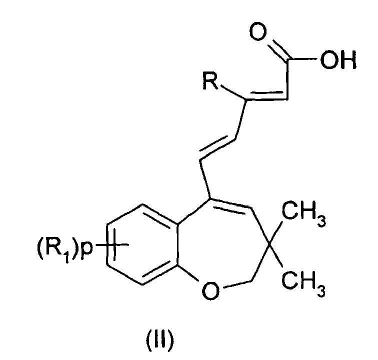 are disclosed in WO00/39113 as useful for treating dyslipidemias, atherosclerosis and diabetes.

In this application, compounds of formula II are prepared according to the following scheme 1 :

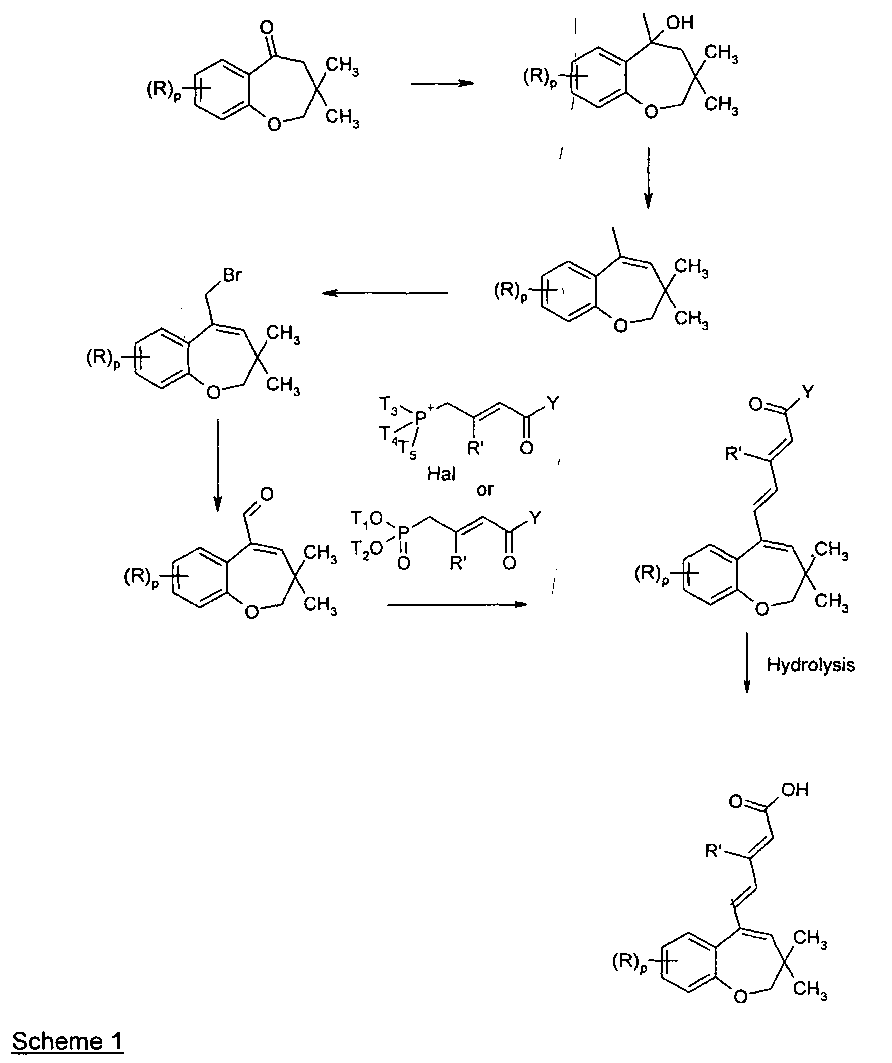

The above synthetic method involves a brominating step by means of NBS; this step may lead to several by-products, in particular in the presence of alkyl substituents on the molecule.

Additionally, the above method involves coupling with phosphonium or phosphonate derivatives of specific formulae:

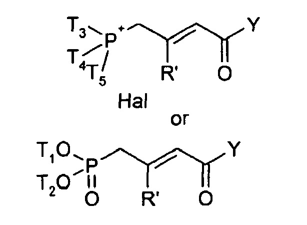

Due to the above specific formulae required, choice and availability of suitable phosphonium and phosphonate species are rather restricted. Further, this may limit the diversity of the structure of compounds of formula (II) which may possibly be prepared according to the above method.

Finally, the above method does not allow preparation of compounds substituted in the alpha-position relative to the acid group.

It now has been found a novel improved synthetic route for preparing the compounds of formula (II) which unexpectedly overcomes the above drawbacks of the prior art method.

Advantageously, the compounds of formula (II) can be obtained by using simpler species, thus leading to potential compounds of greater diversity.

As another advantage, the invention provides an economical and efficient route for preparing the compounds of formula (II).

According to the present invention, compounds of formula (II) are prepared from new compounds of formula (I).

Thus, in one first object, the present invention is related to compounds of general formula (I) :

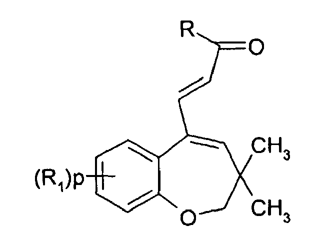

Each of R₁ is independently chosen from a halogen atom; a cyano group; a nitro group; a carboxy group; an optionally halogenated (C₁-C₁₈)alkoxycarbonyl group; an Rₐ-CO-NH- or RₐR_{b}N-CO- group [in which Rₐ and R_{b} independently represent optionally halogenated (C₁-C₁₈)alkyl; a hydrogen atom; (C₆-C₁₀)aryl or (C₆-C₁₀)aryl(C₁-C₅)alkyl (where the aryl parts are optionally substituted by a halogen atom, by an optionally halogenated (C₁-C₅)alkyl group or by an optionally halogenated (C₁-C₅)alkoxy group); (C₃-C₁₂)cycloalkyl optionally substituted by a halogen atom, by an optionally halogenated (C₁-C₅)alkyl group or by an optionally halogenated (C₁-C₅)alkoxy group]; an optionally halogenated (C₁-C₁₈)alkyl group; optionally halogenated (C₁-C₁₈)alkoxy; and (C₆-C₁₀)aryl, (C₆-C₁₀)aryl(C₁-C₅)alkyl, (C₆-C₁₀)aryloxy, (C₃-C₁₂)cyclo-alkyl, (C₃-C₁₂)cycloalkenyl, (C₃-C₁₂)cycloalkyloxy, (C₃-C₁₂)cycloalkenyloxy ; (C₆-C₁₀)aryloxycarbonyl or (C₆-C₁₀)arylcarbonyl;
in which the aryl, cycloalkyl and cycloalkenyl parts are optionally substituted by a halogen atom, by an optionally halogenated (C₁-C₅)alkyl or by an optionally halogenated (C₁-C₅)alkoxy;
p represents 0, 1, 2, 3 or 4;
R represents a hydrogen atom or a (C₁-C₅)alkyl or a (C₆-C₁₀)aryl, each being optionally substituted by a halogen atom, by an optionally halogenated (C₁-C₅)alkyl group or by an optionally halogenated (C₁-C₅)alkoxy group.

The formula (I) encompasses all types of geometric isomers and stereoisomers of the compounds of formula (I) or mixtures thereof.

As used above and throughout the description of the invention, the following terms, unless otherwise indicated, shall be understood to have the following meanings.

"Alkyl" means an aliphatic hydrocarbon group which may be straight or branched, having 1 to 18 carbon atoms in the chain. Preferred alkyl groups have 1 to 12 carbon atoms in the chain.

"Branched alkyl" means that one or more lower alkyl groups such as methyl, ethyl or propyl are attached to a linear alkyl chain.

"Lower alkyl" means an alkyl group with 1 to about 4 carbon atoms in the chain which may be straight or branched.

Exemplary alkyl groups include methyl, ethyl, propyl, isopropyl, butyl, tert-butyl, isobutyl, pentyl, hexyl, heptyl, octyl, nonyl, decyl, undecyl, dodecyl, tridecyl, tetradecyl, pentadecyl, hexadecyl, heptadecyl or octadecyl.

The alkyl group may be substituted by one or more halogen atoms representing thus an "halogenoalkyl" group.

"Halogen atoms" means fluorine, chlorine, bromine or iodine atoms. Preferred are fluorine, chlorine or bromine atoms and more preferred is fluorine atoms.

The "halogenoalkyl" groups may thus refer to "perfluoroalkyl", which means groups corresponding to the formula "-CₙF₂ₙ₊₁" wherein n represents 1 to 18.

Examples of perfluoroalkyl groups are pentafluoroethyl or trifluoro-methyl.

"Alkoxy" means an alkyl-O- group wherein the alkyl group is as herein described. Exemplary alkoxy groups include methoxy, ethoxy, isopropyloxy, butoxy and hexyloxy radicals.

"Cycloalkyl" means a non-aromatic mono- or multicyclic ring system of about 3 to 12 carbon atoms. Preferred ring sizes of the ring system include about 3 to 8 and more preferably 5 to 6 ring atoms. The cycloalkyl is optionally substituted with one or more "ring system substituents" which may be the same or different, and are as defined herein. Exemplary monocyclic cycloalkyl include cyclopropyl, cyclobutyl, cyclopentyl, cyclohexyl, cycloheptyl, cyclooctyl, cyclononyl, cyclodecyl, cycloundecyl, cyclododecyl and the like.

Exemplary multicyclic cycloalkyl include 1-decalyn, norbornyl and the like.

"Cycloalkenyl" means a non-aromatic mono- or multicyclic ring system of about 3 to about 12 carbon atoms, preferably of about 5 to about 10 carbon atoms, and which contain at least one carbon-carbon double bond. Preferred ring size of rings of the ring system includes about 5 to about 6 ring atoms. The cycloalkenyl is optionally substituted with one or more "ring system substituents" which may be the same or different, and are as defined herein. Exemplary monocyclic cycloalkenyl include cyclopentenyl, cyclo-hexenyl, cycloheptenyl and the like. An exemplary multicyclic cycloalkenyl is norbornylenyl.

"Aryl" means an aromatic monocyclic or multicyclic ring system of about 6 to about 10 carbon atoms. The aryl is optionally substituted with one or more "ring system substituents" which may be the same or different and are as defined herein. Exemplary aryl groups include phenyl or naphtyl, or substituted phenyl or substituted naphtyl.

"Alkenyl" means an aliphatic hydrocarbon group containing one or more carbon-carbon double bond and which may be straight or branched, having about 2 to about 12 carbon atoms in the chain, and more preferably about 2 to about 4 carbon atoms in the chain.

"Branched alkenyl" means that one or more lower alkyl or alkenyl groups such as methyl, ethyl or propyl are attached to a linear alkenyl chain. "Lower alkenyl" means about 2 to about 4 carbon atoms in the chain, which may be straight or branched. The alkenyl group may be substituted by one or more halogen atoms. Exemplary alkenyl groups include ethenyl, propenyl, n-butenyl, i-butenyl, 3-methylbut-2-enyl, n-pentenyl, heptenyl, octenyl, cyclohexyl-butenyl and decenyl.

"Aryloxy" means an aryl-O- group wherein the aryl group is as defined herein. Exemplary groups include phenoxy and 2-naphtyloxy.

"Aryloxycarbonyl" means an aryl-O-CO- group wherein the aryl group is as defined herein. Exemplary aryloxycarbonyl groups include phenoxy-carbonyl and naphtoxycarbonyl.

"Arylcarbonyl" refers to an aryl-CO- group wherein the aryl group is as defined herein.

Exemplary arylcarbonyl group includes benzoyl.

The (C₆-C₁₀) aryl, (C₃-C₁₂) cycloalkyl, (C₃-C₁₂) cycloalkenyl are optionally substituted by one or more "ring system substituents".

"Ring system substituents" mean substituents attached to aromatic or non-aromatic ring systems, inclusive of halogen atoms, an optionally halogenated (C₁-C₅) alkyl, or an optionally halogenated (C₁-C₅) alkoxy, halogen, alkyl and alkoxy being as defined herein,

The wording "in which the aryl, cycloalkyl and cycloalkenyl parts are optionally substituted by a halogen atom, by an optionally halogenated (C₁-C₅)alkyl or by an optionally halogenated (C₁-C₅)alkoxy" means that the aryl, cycloalkyl, cycloalkenyl groups are optionally substituted by one or more substituents selected from the group consisting of :
- halogen atoms ;
- alkyl groups optionally substituted by one or more halogen atoms, and
- alkoxy groups optionally substituted by one or more halogen atoms.

The wording "optionally halogenated" means, in the context of the description, optionally substituted by one or more halogen atoms.

Examples of preferred halogen atoms include fluorine, bromine and chlorine atoms.

Examples of optionally substituted halogenated (C₆-C₁₀) arylcarbonyl include the groups ortho, meta or para chlorobenzoyl, or ortho, meta, para-bromobenzoyl group.

Examples of preferred optionally halogenated (C₁-C₁₈) alkyl include notably perfluoroalkyl groups such as trifluoromethyl.

Examples of preferred optionally halogenated (C₁-C₁₈) alkoxy include notably optionally halogenated (C₁-C₆) alkoxy, particularly (C₁-C₄) alkoxy such as methoxy, ethoxy, isopropyloxy, n-butoxy, isobutoxy.

Examples of particularly preferred optionally halogenated (C₆-C₁₀) aryl include notably phenyl.

According to a preferred aspect, each of R₁ independently represents a halogen atom, preferably chlorine or bromine atom; an halogenated (C₁-C₁₈)alkyl group, preferably a perfluoro(C₁-C₁₈)alkyl; a (C₁-C₁₈)alkyl, preferably a methyl or isopropyl group; a optionally halogenated (C₁-C₁₈)alkoxy, preferably a perfluoroalkoxy; an optionally halogenated (C₆-C₁₀)aryl, preferably phenyl or halogenophenyl; and a (C₁-C₁₈) alkoxy group, more preferably a (C₁-C₄) alkoxy group and, most preferably, a methoxy group.

Preferably, p is 1 or 2 and more preferably 1.

R may be located in position 6, 7, 8 or 9 on the benzoxepine structure, as represented hereafter:

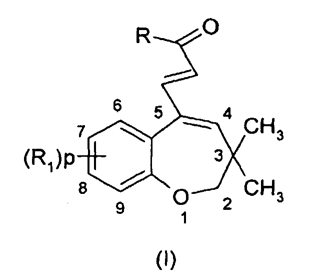

Preferably, R represents a (C₁-C₅) alkyl.

Preferred compounds of formula (I) are chosen from:
(3E)-4-(3,3-dimethyl-7-ethyl-2,3-dihydrobenzoxepin-5-yl)buten-2one ;
(3E)-4-(3,3-dimethyl-7-methoxy-2,3-dihydrobenzoxepin-5-yl)buten-2one ;
(3E)-4-(3,3-dimethyl-7,8-dimethoxy-2,3-dihydrobenzoxepin-5-yl)buten-2one ;
(3E)-4-(3,3-dimethyl-8-chloro-2,3-dihydrobenzoxepin-5-yl)buten-2one ;
(3E)-4-(3,3-dimethyl-7-chloro-2,3-dihydrobenzoxepin-5-yl)buten-2one ;
(3E)-4-(3,3-dimethyl-7-bromo-2,3-dihydrobenzoxepin-5-yl)buten-2one ;
(3E)-4-(3,3-dimethyl-8-chloro-7-fluoro-2,3-dihydrobenzoxepin-5-yl)buten-2one;
(3E)-4-(3,3-dimethyl-7-fluoro-2,3-dihydrobenzoxepin-5-yl)buten-2one ;
(3E)-4-(3,3-dimethyl-7-trifluoromethyl-2,3-dihydrobenzoxepin-5-yl)buten-2one;
(3E)-4-(3,3-dimethyl-7-phenyl-2,3-dihydrobenzoxepin-5-yl)buten-2one ;
(3E)-4-(3,3-dimethyl-7-methyl-2,3-dihydrobenzoxepin-5-yl)buten-2one ;
(3E)-4-(3,3-dimethyl-9-methoxy-2,3-dihydrobenzoxepin-5-yl)buten-2one ;
(3E)-4-(3,3-dimethyl-7,8-dimethyl-2,3-dihydrobenzoxepin-5-yl)buten-2one ;
(3E)-4-(3,3-dimethyl-8-(4-fluorophenyl)-2,3-dihydrobenzoxepin-5-yl)buten-2one ;
(3E)-4-(3,3-dimethyl-8-methoxy-2,3-dihydrobenzoxepin-5-yl)buten-2one ;
(3E)-4-(3,3-dimethyl-7-isopropyl-2,3-dihydrobenzoxepin-5-yl)buten-2one ;
(3E)-4-(3,3-dimethyl-7-trifluoromethoxy-2,3-dihydrobenzoxepin-5-yl)buten-2one ;
(2E)-3-(3,3-dimethyl - 7-methoxy - 2,3-dihydrobenzoxepin-5-yl) - 1-phenyl - propen-1-one ;
(1E)-1-(3,3-dimethyl - 8-methoxy - 2,3-dihydrobenzoxepin-5-yl) -hexen-3-one ;
(1E)-1-(3,3-dimethyl - 7-methoxy - 2,3-dihydrobenzoxepin-5-yl) -octen-3-one ;
(1E)-1-(3,3-dimethyl - 8-methoxy - 2,3-dihydrobenzoxepin-5-yl) -octen-3-one ;
as well as their geometric isomers and stereoisomers or mixtures thereof.

A more preferred compound of formula (I) is (3E)-4-(3,3-dimethyl-7-ethyl-2,3-dihydrobenzoxepin-5-yl)buten-2one (Compound IA) : 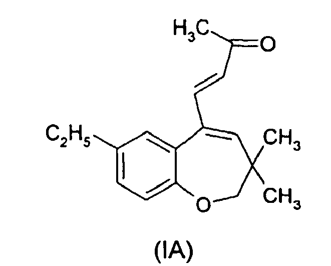 as well as its geometric isomers and stereoisomers or mixtures thereof.

### Methods for Preparing compounds of formula (II) starting from compounds of formula (I)

According to the invention, the compounds of formula (I) are used for the preparation of compounds of formula (II): 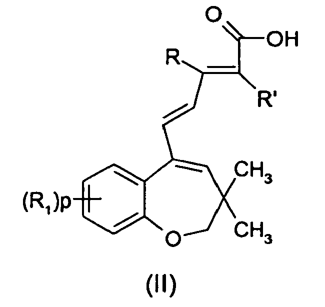 in which R, R₁, p are as defined in formula (I) and R' represents a hydrogen atom or a (C₁-C₅)alkyl or a (C₆-C₁₀)aryl, optionally substituted by a halogen atom, by an optionally halogenated (C₁-C₅)alkyl group or by an optionally halogenated (C₁-C₅)alkoxy group.

Compounds of formula (II), in which R' is a (C₁-C₅)alkyl or a (C₆-C₁₀)aryl, optionally substituted by a halogen atom, by an optionally halogenated (C₁-C₅)alkyl group or by an optionally halogenated (C₁-C₅)alkoxy group could not be prepared according to method disclosed in WO00/39113 and, are, thus, novel.

Thus, in another object, the present invention is directed to a method for preparing compounds of formula (II): 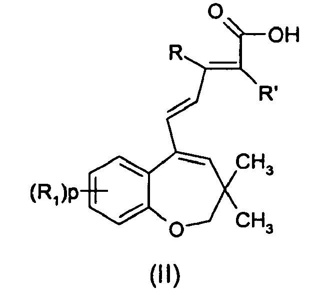 from compounds of formula (I) 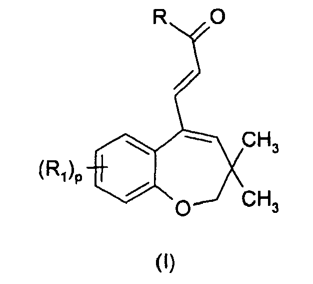 in which R, R₁, p, R' are defined as above,
comprising :
a) carrying out a Wittig reaction or a Horner-Emmons / Wadsworth-Emmons on a compound of formula (I),
b) hydrolyzing the resulting product ; and optionally
c) isolating the obtained compound of formula (II).
In this reaction, R' preferably represents a hydrogen atom.

The reaction of step a) comprises the following step according to scheme 2 :

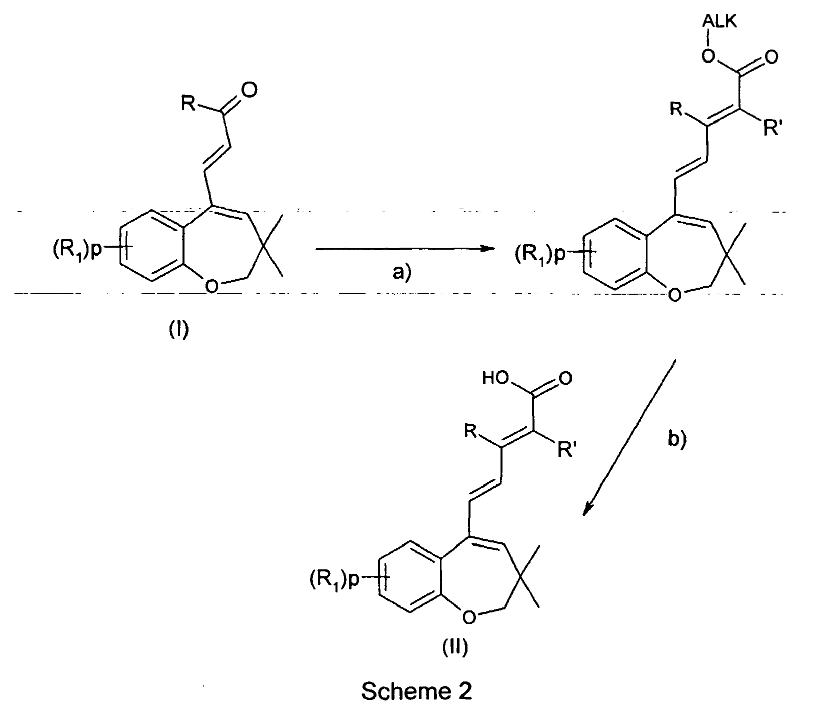

The reaction implemented in scheme 2 can be carried out by applying or adapting either a Wittig reaction or a Horner-Emmons / Wadsworth-Emmons reaction. These reactions are both well-known in the art and typically involve the preparation of a reactive ylid. For any further information on that subject, reference may be made to G. Wittig, U. Schöllkopf, Ber. 87, 1318 (1954) ; G. Wittig, W. Haag, ibid. 88, 1654 (1955) ; L. Horner et al., Ber. 91, 61 (1958) ; idem et al., ibid. 92, 2499 (1959) ; W. S. Wadsworth, Jr., W. D. Emmons, J. Am. Chem. Soc. 83, 1733 (1961).

When the ylid is prepared from a phosphonium salt (compound b), the reaction implemented is a Wittig reaction.

Compounds b have the following ormula: 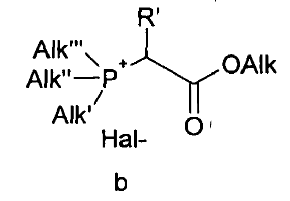 in which Alk, Alk', Alk", Alk''' independently represent a (C₁-C₅) alkyl group and R' is defined as above.

When the ylid is prepared from a phosphonate (compound a), the reaction is called a Horner-Emmons or Wadsworth-Emmons reaction.

Compounds a have the following formula: 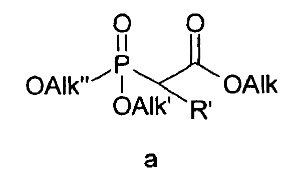 wherein Alk, Alk', Ak" independently represent a (C₁-C₅) alkyl group ; preferably an ethyl group and R' is defined as above.

Under scheme 2, the ylid is prepared by reacting a base either with a compound (a) or with a compound (b). The base used has to be sufficiently strong to remove an hydrogen atom in the alpha-position of the phosphorus.

Typically, the base is selected from the group consisting of alkali metal hydrides, alkali metal carbonates, such as potassium terbutylate, alkali metal amides, (C₁-C₁₀) alkyllithium, and alkali metal alkoxides.

In the context of the invention, alkali metal hydrides such as sodium hydride and potassium hydride, and alkali metal alkoxides such as sodium methoxide, sodium ethoxide and potassium tert-butoxide are particularly preferred. Sodium hydride is particularly preferred.

The reaction of the base on the compounds (a) or (b) is effected in a solution, preferably in an aprotic solvent, and notably in a solvent able to dissolve the phosphonate (a) and respectively the phosphonium salt (b).

Examples of suitable solvents are notably aprotic solvents, such as aromatic hydrocarbons, as for example benzene and toluene, ethers, such as diethylether, dioxane or tetrahydrofuran; aprotic polar solvents such as N,N-dimethylformamide, dimethylsulfoxide, N-methylpyrrolidone or HMPT and mixtures thereof. Toluene is particularly preferred.

The reaction of scheme 2 can take place over a wide range of temperatures, depending on the acidity of the compound (a), respectively (b), which means the ability to remove the hydrogen atom on the alpha-position with regard to the phosphorus. The type of the base used directly influences the choice of the reaction temperature. Thus, the stronger the base is, the lower the reaction temperature is.

When the base is an alkali metal alkoxide, a temperature comprised between -10° and 100°C is generally suitable, preferably between room temperature and the boiling point of the solvent.

A stoichometric amount of basis is generally required in scheme 2 to convert the phosphonate or the phosphonium salt into the corresponding ylid. However, a slight excess of base may be used to ensure the total conversion of the compounds (a) or (b) into the ylid. Thus, the molar ratio of the base relative to the compound (a), respectively (b), is maintained between 0,9 and 1,2, preferably between 0,9 and 1,1, and more preferably between 0,95 and 1. The concentration of the compound (a), respectively (b), in the reaction mixture is not critical according to the invention. The concentration may vary between 0,01 mol/L and 10 mol/L, preferably between 0,1 and 1 mol/L.

According to a preferred embodiment, the ylid resulting from the reaction of the compound (a), respectively (b), with a base is performed before adding the compound of formula (I).

Preferably, the phosphorus ylid is prepared from a phosphonate (a), diethylphosphonoacetate (formula a in which Alk, Alk', Alk" represent ethyl) is preferred.

According to a preferred embodiment, the ylid is prepared by reacting diethylphosphonoacetate with sodium hydride in toluene.

The above reaction of scheme 2 is followed by the step b) of hydrolyzing the obtained ester into the desired acid compound of formula (II).

Preferably, the hydrolysis is performed under alkali conditions followed by acid conditions.

Alkali conditions may be achieved by using sodium hydroxide. Suitable acids for the hydrolysis include inorganic acids, such as hydrochloric acid, sulphuric acid, nitric acid and phosphoric acid ; sulfonic acids such as methanesulfonic acid, ethanesulfonic acid, benzenesulfonic acid and paratoluenesulfonic acid.

Inorganic acids are most preferred and notably hydrochloric acid.

Excess amount of acid is generally used and the amount of acid is for example 5 to 10 moles relative to 1 mole of the ester.

In that context, the mixture is stirred, for example for 0.5 hour to 2 hours and preferably for 1 hour to 1.5 hour.

In that context, it has been found convenient to carry out the reaction at a temperature that does not exceed 40°C, for example from about room temperature to about 40°C.

The time required for each reaction may also vary widely, depending on many factors, notably the reaction temperature and the nature of reagents. However, provided that the reaction is effected under the preferred conditions outlined above, a period from about 0.5 hour to about 2 hours will usually be sufficient for the hydrolysis step.

In optional step c), the compounds thus prepared may be recovered from the reaction mixture by conventional means, for example the compounds may be recovered by distilling of the solvent from the reaction mixture or, if necessary, optionally after distilling of the solvent from the reaction mixture, pouring the residue into water, followed by extraction with a water-immiscible organic solvent and distilling of the solvent from the extract. Additionally, the product can, if desired, be further purified by various well known techniques, such as recrystallization, reprecipitation or the various chromatography techniques, notably column chromatography or preparative thin layer chromatography.

The above phosphonium derivatives of formula b are either commercially available or may be readily prepared by known methods by reacting a phosphine of formula: 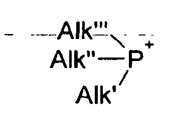 with an halide of formula : 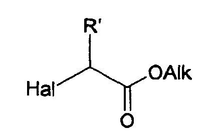 in which Alk, Alk', Alk", Alk''', R' are defined as above and Hal represents an halogen atom.

The above phosphonate derivatives of formula a are either commercially available or may be readily prepared by known methods, such as the Arbuzov method, wherein for instance, when Alk' and Alk" are identical, a phosphate P(OAlk')₃ is reacted with an halide of formula: 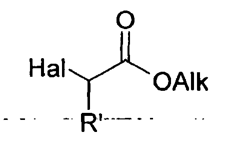 in which Alk, Alk', R' are defined as above and Hal represent an halogen atom.

### Methods for preparing the compounds of formula (I)

The compounds useful according to the invention may be prepared by the application or adaptation of known methods, by which are meant methods used heretofore or described in the literature, for example those described by R. C. Larock in *Comprehensive Organic Transformations,* VCH Publishers, 1989.

The present inventors have also found the following particularly convenient method.

Thus, in a further aspect, the invention provides a method for preparing the compounds of formula (I) comprising :
i) reacting a compound of formula (III): 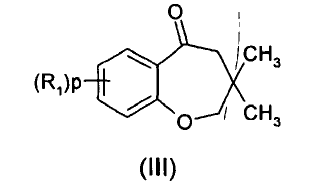 with a perhalogenoalkyl sulfonic derivative;
ii) carrying out a Heck reaction on the resulting product ; and optionally
iii) isolating the obtained compound of formula (I).

This can be illustrated by the following scheme 3 :

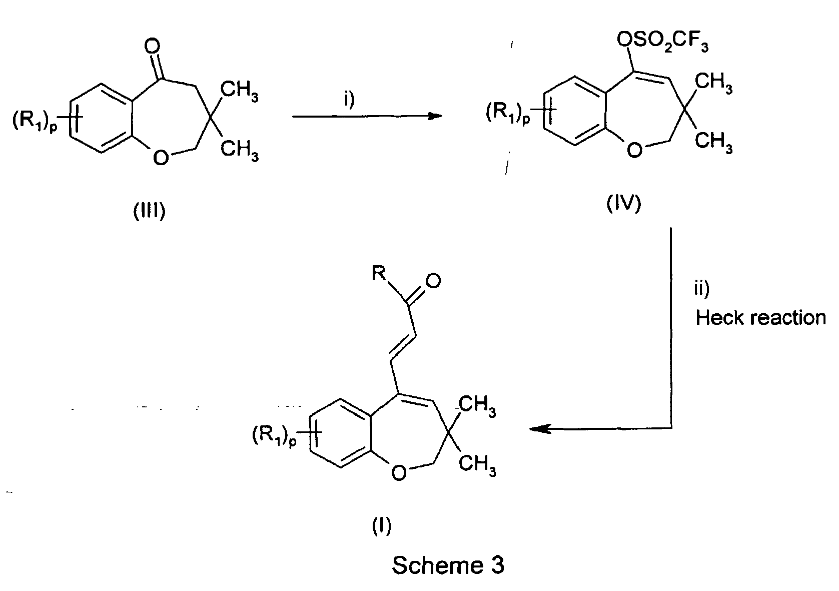

### Step i)

The reaction of step i) consists in converting the compound of formula (III) into the corresponding sulfonic derivative of formula (IV):

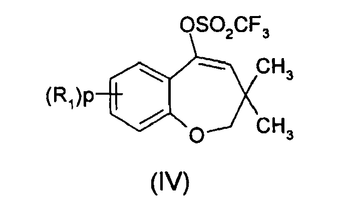

This reaction requires the presence of a perhalogenoalkyl sulfonic derivative. Preferred derivatives include trifluoromethane sulfonic anhydride.

Preferably, the reaction is carried out in the presence of a Lewis acid or a base, such as pyridine, 2,6-diterbutylpyridine; 2,4,6-triterbutylpyridine; 4-Me-2,6-diterbutylpyridine; Na₂CO₃. Pyridine is especially preferred.

The amount of base is generally similar to the amount of the perhalogenoalkyl sulfonic derivative, preferably 0,9 to 1,1 moles relative to 1 mole of said derivative.

There is no particular restriction on the nature of the solvent to be used, provided that it has no adverse effect on the reaction or on the reagent involved.

Examples of suitable solvents include hydrocarbons, such as hexane, cyclohexane, benzene, toluene and xylene; aprotic polar solvents such as N,N-dimethylformamide, N-methylpyrrolidone, dimethylsulfoxide, pyridine, dichloromethane. Of these, dichloromethane are particularly preferred.

The reaction can take place over a wide range of temperatures, and the precise reaction temperature is not critical to the invention. In general, it has been found convenient to carry out the reaction at a temperature from about room temperature (20°C) to 150°C, and more preferably of from 20°C to the boiling point of the solvent.

The molar ratio of the compound of formula (III) relative to the perhalogenoalkyl sulfonic derivative may vary from 2.0 to 6 equivalent, preferably from 4 to 5.

### Step ii)

The reaction under step ii) can be conducted by application or adaptation of the Heck reaction. Details of this well-known reaction can in particular be found in J. Org. Chem. 37, 2320 (1972).

The obtained compound of formula (IV) obtained in step i) is reacted with a compound of formula (V): 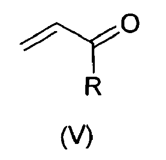 in which R is defined as in formula (I).

This step can be carried out *in situ,* straight after step i), or after isolating compound (IV), or preferably on the crude product after evaporating the solvent.

The molar ration of compound (V) relative to compound of formula (IV) is from 1,2 to 3 equivalents.

The reaction is carried out in the presence of a base.

Examples of suitable basis include alkali metal hydrides, such as lithium hydride, sodium hydride and potassium hydride; (C₁-C₁₀) alkyllithium compounds such as methyllithium, butyllithium, hexyllithium ; alkali metal alkoxides such as sodium methoxide and sodium ethoxide ; organic bases such as triethylamine; and alkali metal carbonates such as potassium carbonate and sodium carbonate. Organic bases such as triethylamine are particularly preferred.

The reaction is palladium catalyzed; suitable catalysts include Pd(OCOCH₃)₂, PdCl₂, Pd(dba)₃, Pd(PPh₃)₄, Pd(PPh₃)Cl₂; Pd(PPh₃)Cl₂ is preferred.

A ligand such as PAr₃, dppp, binap may also be used.

There is no particular restriction on the nature of the solvent to be used, provided that it has no adverse effect on the reaction or on the reagent involved.

Examples of suitable solvents include hydrocarbons, such as hexane, cyclohexane, benzene, toluene and xylene; aprotic polar solvents such as N,N-dimethylformamide, N-methylpyrrolidone, dimethylsulfoxide, pyridine. Of these, dimethylformamide is particularly preferred.

The reaction can take place over a wide range of temperatures, and the precise reaction temperature is not critical to the invention. In general, it has been found convenient to carry out the reaction at a temperature from about room temperature (20°C) to 150°C, preferably of from 20°C to the boiling temperature of the solvent, more preferably 70-80°C.

The reaction can be conducted for a time sufficient to obtain a satisfactory reaction rate, usually between 1 and 10 hours.

### Step iii)

The compounds thus prepared may be recovered from the reaction mixture by conventional means, for example the compounds may be recovered by distilling of the solvent from the reaction mixture or, if necessary, optionally after distilling of the solvent from the reaction mixture, pouring the residue into water, followed by extraction with a water-immiscible organic solvent and distilling of the solvent from the extract. Additionally, the product can, if desired, be further purified by various well known techniques, such as recrystallization, reprecipitation or the various chromatography techniques, notably column chromatography or preparative thin layer chromatography.

The invention is illustrated by the following representative and non-limiting examples :

### Preparative example 1:

### 3,3-dimethyl-5-trifluoromethansulfonyloxy-7-ethyl-2,3-dihydro-benzoxepine (formula (IV) : R = CH₃ and R₁= C₂H₅; (IVA)) :

Trifluoromethane sulfonic anhydride (1.422 kg, 1.10 eq.) is added to a solution of 1 kg, 4.58 moles) of 3,3-dimethyl-7-ethyl-2,3,4,5-tetrahydrobenzoxepin-5-one (IIIA) in dichloromethane containing 0.3999 kg (1.10 eq.) of pyridine. The mixture is heated at reflux for 4 hours.

The reaction mixture is cooled to 20°C and water is added. The organic layer is separated, washed with water and the dichloromethane is evaporated under vacuum. The crude oil (quantitative yield) is used without purification.

### Example 2:

### (3E)-4-(3,3-dimethyl-7-ethyl-2,3-dihydrobenzoxepin-5-yl)-buten-2-one (formula (I) : R = CH₃ and R₁= C₂H₅ ; (IA)) :

The crude oil of example 1 is dissolved in dimethylformamide (3 liters). 0.417 Kg (1.3 eq.) of methylvinylketone, dichloro bis(triphenylphosphine)palladium (0.003 kg) and triethylamine (0.834 kg (1.8 eq.) are added to this solution. The mixture is heated between 70-75°C for 5 hours.

The reaction mixture is cooled, diluted with water (11.00 liters) and toluene (2.20 liters). The organic phase is separated, washed with diluted chlorhydric acid and then water. The toluene phase is washed, filtered on silica and dried. After evaporation of the solvent under vacuum 1.189 kg (yield 96 %) of compound (IA) are obtained.

¹H RMN δ ppm : 1.15 (6H,t) , 2.3 (3H, s) , 2.6 (2H, m) , 3.8 (2H, s) , 6.15 (H, s) , 6.3 (1H, d) , 7.1 (3H, m) , 7.5 (1H, d).

### Preparative example 3 :

### Ethyl ester of (2E,4E)-5-(3,3-dimethyl-7-ethyl-2,3-benzoixepin-5-yl)-3-methylpenta-2,4-dienoic acid (R = CH₃ and R₁= C₂H₅)

1,658 kg (2 eq.) of ethyle diethylphosphonoacetate are added at room temperature to a suspension of 0.288 kg (1.95 eq.) of sodium hydride in toluene. After 1 hour, 1 kg (3.699 moles) of compound (IA) are added and the mixture is heated at 55-60°C for 3 hours (the reaction is followed by TLC).

Water (2 liters) is added. The aqueous phase is discarded and the organic phase is filtered on silica. Toluene is then distillated under vacuum at a temperature up to 70°C and the crude (VA) is used without further purification for the preparation of compound (IIIA).

### Example 4 :

### (2E,4E)-5-(3,3-dimethyl-7-ethyl-2,3-benzoxepin-5-yl)-3-methylpenta-2,4-dienoic acid (formula (II) : R = CH₃ and R₁= C₂H₅; (IIA))

The crude compound (VA) is put into a mixture of ethanol (5 liters) and water (5 liters). A solution of 0.480 liters of concentrated sodium hydroxide is added and then the mixture is heated at reflux for 2 hours (reaction followed by TLC). After partial evaporation at atmospheric pressure, the aqueous phase is washed twice with heptane. After acidification at Ph<2 with chlorhydric acid, the aqueous phase is extracted with a mixture of heptane (4 liters) and toluene (0.2 liters). The organic phase is washed with water and partially evaporated at atmospheric pressure. Isopropanol is added, treated with charcoal and after cooling crystallized compound (IIA) is filtered. The crude compound is purified in methanol/water (90/10). The crystallized (IIA) is filtered at -10°C, washed with aqueous ethanol and dried at 65°C to give (VA), yield =45% identical to compound 35B of EP 1140893.

## Claims

1. Compounds of general formula (I) : 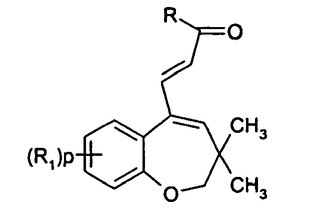
each of R₁ is independently chosen from a halogen atom; a cyano group; a nitro group; a carboxy group; an optionally halogenated (C₁-C₁₈)alkoxycarbonyl group; an Rₐ-CO-NH- or RₐR_{b}N-CO- group [in which Rₐ and R_{b} independently represent optionally halogenated (C₁-C₁₈)alkyl; a hydrogen atom; (C₆-C₁₀)aryl or (C₆-C₁₀)aryl(C₁-C₅)alkyl (where the aryl parts are optionally substituted by a halogen atom, by an optionally halogenated (C₁-C₅)alkyl group or by an optionally halogenated (C₁-C₅)alkoxy group); (C₃-C₁₂)cycloalkyl optionally substituted by a halogen atom, by an optionally halogenated (C₁-C₅)alkyl group or by an optionally halogenated (C₁-C₅)alkoxy group]; an optionally halogenated (C₁-C₁₈)alkyl group; optionally halogenated (C₁-C₁₈)alkoxy; and (C₆-C₁₀)aryl, (C₆-C₁₀)aryl(C₁-C₅)alkyl, (C₆-C₁₀)aryloxy, (C₃-C₁₂)cyclo-alkyl, (C₃-C₁₂)cycloalkenyl, (C₃-C₁₂)cycloalkyloxy, (C₃-C₁₂)cycloalkenyloxy ; (C₆-C₁₀)aryloxycarbonyl or (C₆-C₁₀)arylcarbonyl ;
in which the aryl, cycloalkyl and cycloalkenyl parts are optionally substituted by a halogen atom, by an optionally halogenated (C₁-C₅)alkyl or by an optionally halogenated (C₁-C₅)alkoxy;
p represents 0, 1, 2, 3 or 4;
R represents a hydrogen atom or a (C₁-C₅)alkyl or a (C₆-C₁₀)aryl, each being optionally substituted by a halogen atom, by an optionally halogenated (C₁-C₅)alkyl group or by an optionally halogenated (C₁-C₅)alkoxy group.
as well as their geometric isomers and stereoisomers of the compounds of formula (I) or mixtures thereof.

2. Compounds of formula (I) according to claim 1, wherein each of R₁ independently represents a halogen atom; an halogenated (C₁-C₁₈)alkyl group; a (C₁-C₁₈)alkyl; a optionally halogenated (C₁-C₁₈)alkoxy; an optionally halogenated (C₆-C₁₀)aryl; and a (C₁-C₁₈) alkoxy group.

3. Compounds according to claim 1 or 2 wherein each of R₁ independently represents a chlorine or bromine atom, a perfluoro(C₁-C₁₈)alkyl, a methyl or isopropyl group, a perfluoroalkoxy, a phenyl or halogenophenyl, a methoxy group.

4. Compounds according to claim 1, 2 or 3 wherein each of R₁ independently represents methoxy group.

5. Compounds according to anyone of the preceding claims, wherein p is 1 or 2.

6. Compounds according to anyone of the preceding claims, wherein R is a (C₁-C₅) alkyl group.

7. Compounds according to anyone of the preceding claims chosen from:
(3E)-4-(3,3-dimethyl-7-ethyl-2,3-dihydrobenzoxepin-5-yl)buten-2one ;
(3E)-4-(3,3-dimethyl-7-methoxy-2,3-dihydrobenzoxepin-5-yl)buten-2one ;
(3E)-4-(3,3-dimethyl-7,8-dimethoxy-2,3-dihydrobenzoxepin-5-yl)buten-2one ;
(3E)-4-(3,3-dimethyl-8-chloro-2,3-dihydrobenzoxepin-5-yl)buten-2one ;
(3E)-4-(3,3-dimethyl-7-chloro-2,3-dihydrobenzoxepin-5-yl)buten-2one ;
(3E)-4-(3,3-dimethyl-7-bromo-2,3-dihydrobenzoxepin-5-yl)buten-2one ;
(3E)-4-(3,3-dimethyl-8-chloro-7-fluoro-2,3-dihydrobenzoxepin-5-yl)buten-2one;
(3E)-4-(3,3-dimethyl-7-fluoro-2,3-dihydrobenzoxepin-5-yl)buten-2one ;
(3E)-4-(3,3-dimethyl-7-trifluoromethyl-2,3-dihydrobenzoxepin-5-yl)buten-2one;
(3E)-4-(3,3-dimethyl-7-phenyl-2,3-dihydrobenzoxepin-5-yl)buten-2one ;
(3E)-4-(3,3-dimethyl-7-methyl-2,3-dihydrobenzoxepin-5-yl)buten-2one ;
(3E)-4-(3,3-dimethyl-9-methoxy-2,3-dihydrobenzoxepin-5-yl)buten-2one ;
(3E)-4-(3,3-dimethyl-7,8-dimethyl-2,3-dihydrobenzoxepin-5-yl)buten-2one ;
(3E)-4-(3,3-dimethyl-8-(4-fluorophenyl)-2,3-dihydrobenzoxepin-5-yl)buten-2one ;
(3E)-4-(3,3-dimethyl-8-methoxy-2,3-dihydrobenzoxepin-5-yl)buten-2one ;
(3E)-4-(3,3-dimethyl-7-isopropyl-2,3-dihydrobenzoxepin-5-yl)buten-2one ;
(3E)-4-(3,3-dimethyl-7-trifluoromethoxy-2,3-dihydrobenzoxepin-5-yl)buten-2one ;
(2E)-3-(3,3-dimethyl-7-methoxy-2,3-dihydrobenzoxepin-5-yl)-1-phenyl-propen-1-one ;
(1E)-1-(3,3-dimethyl-8-methoxy-2,3-dihydrobenzoxepin-5-yl)-hexen-3-one ;
(1E)-1-(3,3-dimethy)-7-methoxy-2,3-dihydrobenzoxepin-5-yl)-octen-3-one :
(1E)-1-(3,3-dimethy) - 8-methoxy - 2,3-dihydrobenzoxepin-5-yl)-octen-3-one ;
as well as their geometric isomers and stereoisomers or mixtures thereof.

8. Compounds according to anyone of the preceding claims which is:
(3E)-4-(3,3-dimethyl-7-ethyl-2,3-dihydrobenzoxepin-5-yl)buten-2one.

9. Process of preparation of compounds of formula (I) according to anyone of the preceding claims comprising :
(i) reacting a compound of formula (III): 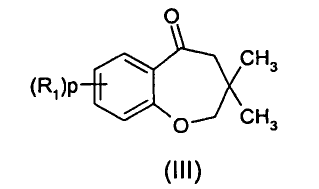 with a perhalogenoalkyl sulfonic derivative;
(ii) carrying out a Heck reaction on the resulting product.

10. Process according to claim 9, wherein step i) comprises converting the compound of formula (III) into the corresponding sulfonic derivative of formula (IV): 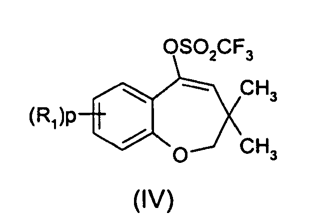 in the presence of a perhalogenoalkyl sulfonic derivative.

11. Process according to claim 9 or 10, wherein said derivative is trifluoromethane sulfonic anhydride.

12. Process according to anyone of claims 9 to 11, wherein step i) is conducted in the presence of a Lewis acid or a base.

13. Process according to claim 12, wherein said Lewis acid or base is chosen from pyridine, 2,6-diterbutylpyridine; 2,4,6-triterbutylpyridine; 4-Me-2,6-diterbutylpyridine; Na₂CO₃.

14. Process according to claim 12 or 13, wherein said Lewis acid or base is pyridine.

15. Process according to anyone of claims 9 to 14, wherein step ii) is carried out according to a Heck reaction.

16. Process according to claim 15, wherein said reaction is conducted with a compound of formula (V): 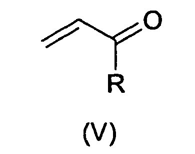 in which R is defined as in formula (I).

17. Process according to anyone of claims 9 to 16 wherein step ii) is carried out *in situ,* straight after step i), or after isolating compound (IV), or on the crude product of step i) after evaporating the solvent.

18. Process according to anyone of claims 15 to 17, wherein step ii) is carried out in the presence of a base.

19. Process according to anyone of claims 15 to 18, wherein step ii) is carried out in the presence of a palladium catalyst.

20. Process according to claim 19, wherein said catalyst is Pd(PPh₃)Cl₂.

21. Process according to anyone of claims 9 to 20 which further comprises the step of isolating the obtained compound of formula (I).

22. Process of preparation of a compound of formula (II): 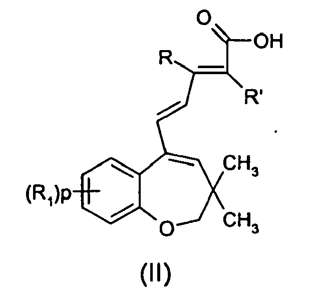 in which R, R₁, p are defined as in anyone of claims 1 to 7 and R' represents a hydrogen atom or a (C₁-C₅)alkyl or a (C₆-C₁₀)aryl, each being optionally substituted by a halogen atom, by an optionally halogenated (C₁-C₅)alkyl group or by an optionally halogenated (C₁-C₅)alkoxy group comprising :
a) carrying out a Wittig reaction or a Horner-Emmons / Wadsworth-Emmons on a compound of formula (I), as defined in anyone of claims 1 to 8,
b) hydrolyzing the resulting product.

23. Process according to claim 22, wherein R' represents a hydrogen atom.

24. Process according to claim 22 or 23, wherein step a) is carried out with a compound of formula b 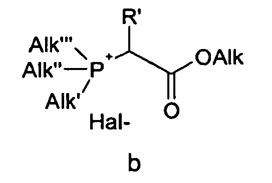 in which Alk, Alk', Alk", Alk"' independently represent a (C₁-C₅) alkyl group and R' is defined as in claim 22, or of formula a: 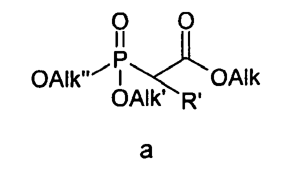 wherein Alk, Alk', Ak" independently represent a (C₁-C₅) group and R' is defined as in claim 22.

25. Process according to anyone of claims 22 to 24, wherein a base is reacted either with a compound (a) or with a compound (b).

26. Process according to claim 25, wherein said base is chosen from alkali metal hydrides and alkali metal alkoxides.

27. Process according to anyone of claims 21 to 26, wherein step b) comprises the step of hydrolyzing the obtained product into the desired acid compound of formula (II).

28. Process according to claim 27, wherein said hydrolysis is conducted under acid conditions.

29. Process according to anyone of claims 22 to 28, which further comprises the step of isolating the obtained compound of formula (II).

## Patentansprüche

1. Verbindungen der allgemeinen Formel (I): 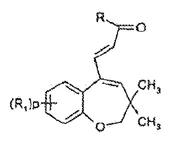
wobei jeweils R₁ unabhängig aus einem Halogenatom; einer Cyanogruppe; einer Nitrogruppe: einer Carboxygruppe; einer wahlweise halogenhaltigen (C₁-C₁₈)Alkoxycarbonylgruppe; einer Rₐ-CO-NH- oder RₐR_{b}N-CO-Gruppe [bei welcher Rₐ und R_{b} unabhängig wahlweise halogenhaltiges (C₁-C₁₈)alkyl; ein Wasserstoffatom; (C₆-C₁₀)Aryl oder (C₆-C₁₀)Aryl(C₁-C₅)Alkyl (wobei die Arylteile wahlweise durch ein Halogenatom, durch eine wahlweise halogenhaltige (C₁-C₅)Alkylgruppe oder durch eine wahlweise halogenhaltige (C₁-C₅)Alkoxygruppe substituiert sind) darstellen; wobei (C₃-C₁₂)Cycloalkyl wahlweise durch ein Halogenatom, durch eine wahlweise halogenhaltige (C₁-C₅)Alkylgruppe oder durch eine wahlweise halogenhaltige (C₁-C₅)Alkoxygruppe substituiert sind]; aus einer wahlweise halogenhaltigen (C₁-C₁₈)Alkylgruppe; wahlweise halogenhaitigern (C₁-C₁₈)Alkoxy sowie (C₆-C₁₀)Aryl, (C₆-C₁₀)Aryl(C₁-C₅)Alkylgruppe; wahlweise halogesihraltigem (C₁-C₁₈)Alkoxy und (C₆-C₁₀)Aryl(C₁-C₅Alkyl, (C₆-C₁₀)Aryloxy, (C₃-C₁₂)Cycloalkyl, (C₃-C₁₂)Cycloalkenyl; (C₃-C₁₂)Cycloalkyloxy, (C₃-C₁₂)Cycloalkenyloxy: (C₆-C₁₀)Aryloxycarbonyl oder (C₆-C₁₀)Arylcarbonyl ausgewählt ist;
wobei die Aryl-, Cycloalkyl- und Cycloalkenylteile wahlweise durch ein Halogenatom, durch ein wahlweise halogenhaltiges (C₁-C₅)Alkyl oder durch ein wahlweise halogenhaltiges C₁-C₅)Alkoxy substituiert sind;
p 0, 1, 2, 3 oder 4 darstellt;
R ein Wasserstoffatom oder ein (C₁-C₅)Alkyl oder ein (C₆-C₁₀)Aryl darstellt, wobei jedes wahlweise durch ein Halogenatom, durch eine wahlweise halogenhaltiges (C₁-C₅)Alkylgruppe oder durch eine wahlweise halogenhaltige (C₁-C₅)Alkoxygruppe substituiert ist,
sowohl als auch deren geometrischen Isomere und Stereoisomere der Verbindungen der Formel (I) oder deren Mischungen.

2. Verbindungen der Formel (I) nach Anspruch 1, wobei jeweils R₁ unabhängig ein Hatogenatom; eine halogenhaltige (C₁-C₁₈)Alkylgruppe; ein (C₁-C₁₈)Alkyl; ein wahlweise halogenhaltiges (C₁-C₁₈)Alkoxy; ein wahlweise halogenhaltiges (C₆-C₁₀)Aryl sowie eine (C₁-C₁₈)Alkoxygruppe darstellt.

3. Verbindungen nach Anspruch 1 oder 2, wobei jeweils R₁ unabhängig ein Chlor- oder Bromatom, ein Perfluor(C₁-C₁₈)alkyl, eine Methyl- oder Isopropylgruppe, ein Perfluoralkoxy, ein Phenyl oder Halogenphenyl, eine Methoxygruppe darstellt.

4. Verbindungen nach Anspruch 1, 2 oder 3, wobei jeweils R₁ unabhängig eine Methoxygruppe darstellt.

5. Verbindungen nach einem der vorstehende Ansprüche, wobei p 1 oder 2 ist.

6. Verbindungen nach einem der vorstehenden Ansprüche, wobei R eine (C₁-C₅)Alkylgruppe ist.

7. Verbindungen nach einem der vorstehenden Ansprüche, ausgewählt aus:
(3E)-4-(3,3-Dimethyl-7-Ethyl-2,3-Dihydrobenzoxepin-5-yl)buten-2eins ;
(3E)-4-(3,3-Dimethyyl-7-Methoxy-2,3-Dihydrobenzoxepin-5-yl)buten-2eins ;
(3E)-4-(3,3-Dimethyl-7,8-Dimethoxy-2,3-Dihydrobenzoxepin-5-yl)buten-2eins :
(3E)-4-(3,3-Dimethyl-8-Chlor-2,3-Dihydrobenzoxepin-5-yl)buten-2eins ;
(3E)-4-(3,3-Dimethyl-7-Chlor-2,3-Dihydrobenzoxepin-5-yl)buten-2eins ;
(3E)-4-(3,3-Dimethyl-7-Brom-2,3-Dihydrobenzoxepin-3-yl)buten-2eins ;
(3E)-4-(3,3-Dimethyl-8-Chlor-7-Fluor-2,3-Dihydrobenzoxepin-5-yl)buten-2eins;
(3E)-4-(3,3-Dimethyl-7-fluor-2,3-Dihydrobenzoxepin-5-yl)buten-2eins ;
(3E)-4-(3,3-Dimethyl-7-Trifluormethyl-2,3-Dihydrobenzoxepin-5-yl)buten-2eins ;
(3R)-4-(3,3-Dimethyl-7-Phenyl-2,3-Dihydrobenzoxepin-5-yl)buten-2eins ;
(3E)-4-(3,3-Dimethyl-7-Methyl-2,3-Dihydrobenzoxepin-5-yl)buten-2eins ;
(3E)-4-(3,3-Dimethyl-9-Methoxy-2,3-Dihydrobenzoxepin-5-yl)buten-2eins ;
(3E)-4-(3,3-Dimethyl-7,8-Dimethyl-2,3-Dihydrobenzoxepin-5-yl)buten-2eins ;
(3E)-4-(3,3-Dimethyl-8-(4-Fluorphenyl)-2,3-Dihydrobenzoxepin-5-yl)buten-2eins ;
(3E)-4-(3,3-Dimethyl-8-Methoxy-2,3-Dihydrobenzoxepin-5-yl)buten-2eins;
(3E)-4-(3,3-Dimethyl-7-isopropyl-2,3-Dihydrobenzoxepin-5-yl)buten-2eins ;
(3E)-4-(3,3-Dimethyl-7-Trifluormethoxy-2,3-Dihydrobenzoxepin-5-yl)buten-2eins ;
(2E)-3-(3,3-Dimethyl-7-Methoxy-2,3-Dihydrobenzoxepin-5-yl)-1-Phenyl-Propen-1-eins ;
(1E)-1-(3,3-Dimethyl-8-Methoxy-2,3-Dihydrobenzoxepin-5-yl)-Hexen-3-eins ;
(1E)-1-(3,3-Dimethyl-7-Methoxy-2,3-Dihydrobenzoxepin-5-yl)-Octen-3-eins ;
(1E)-1-(3,3-Dimethyl-8-Methoxy-2,3-Dihydrobenzoxepin-5-yl)-Octen-3-eins ;
sowie deren geometrischen Isomere und Stereoisomere oder deren Mischungen.

8. Verbindungen nach einem der vorstehenden Ansprüche, das heißt:
(3E)-4-(3,3-Dimethyl-7-Ethyl-2,3-Dihydrobenzoxepin-5-yl)buten-2eins.

9. Verfahren zur Herstellung von Verbindungen nach Formel (I) nach einem der vorstehenden Ansprüche, umfassend:
(i) das Eingehen einer Reaktion mit einer Verbindung der Forme (1H): 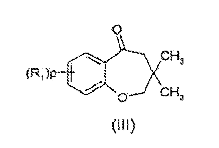 zusammen mit einem Perhalogenalkylsulfonderivat;
(ii) das Durchführen einer Heck-Reaktion am resultierenden Produkt.

10. Verfahren nach Anspruch 9, wobei Schritt i) die Umsetzung der Verbindung der Formel (III) in ein entsprechendes Sulfonderivat der Formel (IV) 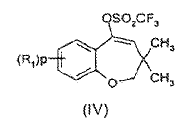 in Anwesenheit eines Perhalogenalkylsulfonderivats umfasst.

11. Verfahren nach Anspruch 9 oder 10, wobei das genannte Derivat Trifluormethansulfonanhydrid ist.

12. Verfahren nach einem der Anspruche 9 bis 11, wobei Schritt i) in Anwesenheit einer Lewissäure oder-base ausgeführt wird.

13. Verfahren nach Anspruch 12, wobei die genannte Lewissäure oder-base ausgewählt ist aus Pyridin, 2,5-Diterbutylpyridin; 2,4,6-Triterbutylpyridin; 4-Me-2,6-Diterbutylpyridin; Na₂CO₃.

14. Verfahren nach Anspruch 12 oder 13, wobei die genannte Lewissäure oder -base Pyridin ist.

15. Verfahren nach einem der Ansprüche 9 bis 14, wobei Schritt ii) gemäß einer Heck-Reaktion aufgeführt wird.

16. Verfahren nach Anspruch 15, wobei die genannte Reaktion mit einer Verbindung der Formel (V) durchgeführt wird: 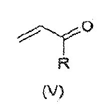 wobei R wie in Formel (I) definiert ist.

17. Verfahren nach einem der Anspruche 9 bis 16, wobei Schritt ii) *in situ* unmittelbar nach Schritt i) oder nach dem isolieren der Verbindung (IV) oder am Rohprodukt von Schritt i), nachdem das Lösungsmittel evaporiert ist, ausgeführt wird.

18. Verfahren nach einem der Ansprüche 15 bis 17, wobei Schritt ii) in Anwesenheit einer Base ausgeführt wird.

19. Verfahren nach einem der Ansprüche 15 bis 18, wobei Schritt ii) in Anwesenheit eines Palladiumkatalysators ausgeführt wird.

20. Verfahren nach Anspruch 19, wobei der genannte Katalysator Pd(PPh₃)Cl₂ ist.

21. Verfahren nach einem der Ansprüche 9 bis 20, ferner umfassend den Schritt des Isolierens der erhaltenen Verbindung der Formel (I).

22. Verfahren zur Herstellung einer Verbindung der Formel (II): 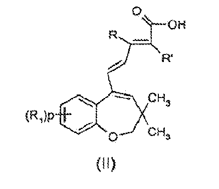 wobei R, R₁, p wie in einem der Ansprüche 1 bis 7 definiert sind und R' ein Wasserstoffatom oder ein (C₁-C₅)Alkyl oder ein (C₆-C₁₀)Aryl darstellt, wobei jedes wahlweise durch ein Halogenatom, durch eine wahlweise halogenhaltige (C₁-C₅)Alkylgruppe oder durch eine wahlweise halogenhaltige (C₁-C₅)Alkoxygruppe substituiert ist, umfassend:
a) das Ausführen einer Wittig-Reaktion oder einer Horner-Emmons-Reaktion/Wadsworth-Emmons-Reaktion an einer Verbindung der Formel (I) wie in einem der Ansprüche 1 bis 8 definiert,
b) das Hydrolysieren des resultierenden Produkts.

23. Verfahren nach Anspruch 22, wobei R' ein Wasserstoffatom darstellt

24. Verfahren nach Anspruch 22 oder 23, wobei Schritt a) mit einer Verbindung der Formel b ausgeführt wird 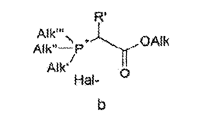 wobei Alk, Alk', Alk", Alk'" unabhängig eine (C₁-C₅)Alkylgruppe darstellen und R' wie in Anspruch 22 definiert ist, oder der Formel a: 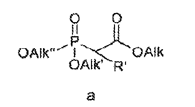 wobei Alk, Alk', Alk" unabhängig eine (C₁-C₅)-Gruppe darstellen und R' wie in Anspruch 22 definiert ist.

25. Verfahren nach einen der Ansprüche 22 to 24, wobei eine Base entweder mit einer Verbindung (a) oder mit einer Verbindung (b) in Reaktion gebracht wird.

26. Verfahren nach einem der Anspruche 25, wobei die genannte Base ausgewählt ist aus Alkalimetallhydriden und Alkalimetallakoxiden.

27. Verfahren nach einem der Anspruche 21 bis 26, wobei Schritt b) den Schritt des Hydrolysierens des erhaltenen Produkts in eine gewünschte Säureverbindung der Formel (II) umfasst.

28. Verfahren nach Anspruch 27, wobei die genannte Hydrolyse unter Säurebedingungen durchgeführt wird.

29. Verfahren nach einem der Anspruche 22 bis 28, ferner umfassend den Schritt des Isolierens der erhaltenen Verbindung der Formel (II).

## Revendications

1. Composés de formule générale (I) : 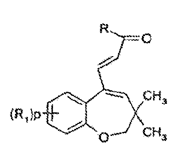 dans lesquels :
chaque R₁ est indépendamment choisi parmi un atome d'halogène ; un groupe cyano ; un groupe nitro; un groupe carboxy; un groupe alcoxycarbonyle (en C₁ à C₁₈) facultativement halogéné ; un groupe Rₐ-CO-NH- ou RₐR_{b}N-CO- [dans lequel Rₐ et R_{b} représentent indépendamment un alkyle (en C₁ à C₁₈) facultativement halogéné ; un atome d'hydrogène ; un aryle (en C₆ à C₁₀) ou aryl (en C₆ à C₁₀)-alkyle (en C₁ à C₅) (où les parties aryle sont facultativement substituées par un atome d'halogène, par un groupe alkyle (en C₁ à C₅) facultativement halogéné ou par un groupe alcoxy (en C₁ à C₅) facultativement halogéné) ; un cycloalkyle (en C₃ à C₁₂) facultativement substitué par un atome d'halogène, par un groupe alkyle (en C₁ à C₅) facultativement halogéné ou par un groupe alcoxy (en C₁ à C₅) facultativement halogéné]; un groupe alkyle (en C₁ à C₁₈) halogéné ; un groupe alcoxy (en C₁ à C₁₈) facultativement halogène ; et un aryle (en C₆ à C₁₀), aryl (en C₆ à C₁₀)-alkyle (en C₁ à C₅), aryloxy (en C₆ à C₁₀), cyclo-alkyle (en C₃ à C₁₂), cycloalcényle (en C₃ à C₁₂), cycloalkyloxy (en C₃ à C₁₂), cycloalcényloxy (en C₃ à C₁₂) ; aryloxycarbonyle (en C₆ à C₁₀) ou arylcarbonyle (en C₆ à C₁₀);
où les parties aryle, cycloalkyle et cycloalcényle sont facultativement substituées par un atome d'halogène, par un alkyle (en C₁ à C₅) facultativement halogéné ou par un alcoxy (en C₁ à C₅) facultativement halogéné ;
p représente 0, 1, 2, 3 ou 4 ;
R représente un atome d'hydrogène ou un alkyle (en C₁ à C₅) ou un aryle (en C₆ à C₁₀), chacun étant facultativement substitué par un atome d'halogène, par un groupe alkyle (en C₁ à C₅) facultativement halogéné ou par un groupe alcoxy (en C₁ à C₅) facultativement halogéné, ainsi que leurs isomères et stéréoisomères géométriques des composés de formule (1) ou leurs mélanges.

2. Composés de formule (1) selon la revendication 1, dans lesquels chaque R₁ représente indépendamment un atome d'halogène ; un groupe alkyle (en C₁ à C₁₈) halogéné ; un alkyle (en C₁ à C₁₈) ; un alcoxy (en C₁ à C₁₈) facultativement halogéné; un aryle (en C₆ à C₁₀) facultativement halogéné ; et un groupe alcoxy (en C₁ à C₁₈).

3. Composés selon la revendication 1 ou 2, dans lesquels chaque R₁ représente indépendamment un atome chlore ou de brome, un perfluoro-alkyle (en C₁ à C₁₈), un groupe méthyle ou isopropyle, un perfluoroalcoxy, un phényle ou halogénophényle, un groupe méthoxy.

4. Composés selon la revendication 1, 2 ou 3, dans lesquels chaque R₁ représente indépendamment un groupe méthoxy.

5. Composés selon l'une quelconque des revendications précédentes, dans lesquels p vaut 1 ou 2.

6. Composés selon l'une quelconque des revendications précédentes, dans lesquels R est un groupe alkyle (en C₁ à C₅).

7. Composés selon l'une quelconque des revendications précédentes, choisis parmi :
la (3E)-4-(3,3-diméthyl-7-ethyl-2,3-dihydrobenzoxépin-5-yl)butén-2-one ;
la (3E)-4-(3,3-diméthyl-7-méthoxy-2,3-dihydrobenzoxépin-5-yl)butén-2-one ;
la (3E)-4-(3,3-diméthyl-7,8-diméthoxy-2,3-dihydrobenzoxépin-5-yl)butén-2-one ;
la (3E)-4-(3,3-dimethyl-8-chloro-2,3-dihydrobenzoxépin-5-yl)butén-3-one ;
la (3E)-4-(3,3-dimethyl-7-chloro-2,3-dihydrobenzoxépin-5-yl)butén-2-one ;
la (3E)-4-(3,3-diméthyl-7-bromo-2,3-dihydrobenzoxépin-5-yl)butén-2-one ;
la (3E)-4-(3,3-diméthyl-8-bromo-7-fluoro-2,3-dihydrobenzoxépin-5-yl)butén-2-one ;
la (3E)-4-(3,3-diméthyl-7-fluoro-2,3-dihydrobenzoxépin-5-yl)butén-2-one ;
la (3E)-4-(3,3-diméthyl-7-trifluorométhyl-2,3-dihydrobenzoxépin-5-yl)butén-2-one ;
la (3E)-4-(3,3-diméthyl-7-phényl-2,3-dihydrobenzoxépin-5-yl)butén-2-one ;
la (3E)-4-(3,3-diméthyl-7-méthyl-2,3-dihydrobenzoxépin-5-yl)butén-2-one ;
la (3E)-4-(3,3-diméthyl-9-méthoxy-2,3-dihydrobenzoxépin-5-yl)butén-2-one ;
la (3E)-4-(3,3-diméthyl-7,8-diméthyl-2,3-dihydrobenzoxépin-5-yl)butén-2-one ;
la (3E)-4-(3,3-diméthyl-8-(4-fluorophenyl)-2,3-dihydrobenzoxépin-5-yl)butén-2-one ;
la (3E)-4-(3,3-diméthyl-8-méthoxy-2,3-dihydrobenzoxépin-5-yl)buten-2-one ;
la (3E)-4-(3,3-dimethyl-7-isopropyl-2,3-dihydrobenzoxépin-5-yl)butén-2-one ;
la (3E)-4-(3,3-diméthyl-7-trifluorométhoxy-2,3-dihydrobenzoxépin-5-yl)butén-2-one ;
la (2E)-3-(3,3-diméthyl-7-méthoxy-2,3-dihydrobenzoxépin-5-yl)-1-phényl-propén-1-one ;
la (1E)-1-(3,3-diméthyl-8-méthoxy-2,3-dihydrobenzoxépin-5-yl)-hexén-3-one ;
la (1E)-1-(3,3-diméthyl-7-méthoxy-2,3-dihydrobenzoxépin-5-yl)-octén-3-one ;
la (1)-1-(3,3-diméthyl-8-méthoxy-2,3-dihydrobenzoxépin-5-yl)-octén-3-one ;
ainsi que leurs isomères et stéréoisomères géométriques ou leurs mélanges.

8. Composés selon l'une quelconque des revendications précédentes qui est :
la (3E)-4-(3,3-dimethyl-7-éthyl-2,3-dihydrobenzoxépin-5-yl)butén-2-one.

9. Procédé de préparation des composés de formule (I) selon l'une quelconque des revendications précédentes comprenant les étapes consistant à:
(i) faire réagir un composé de formule (III): 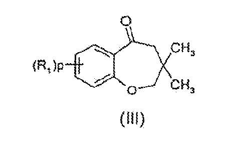 avec un dérivé perhalogénoalkyle sulfonique ;
(ii) réaliser une réaction de Heck sur le produit résultant.

10. Procédé selon la revendication 9, dans lequel l'étape (i) comprend la conversion du composé de formule (III) en le dérivé sulfonique correspondant de formule (IV): 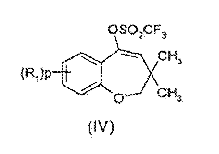 en présence d'un dérivé perhalogénoalkyle sulfonique.

11. Procédé selon la revendication 9 ou 10, dans lequel ledit dérivé est un anhydride de trifluorométhane sulfonique.

12. Procédé selon l'une quelconque des revendications 9 à 11, dans lequel l'étape (i) est conduite en présence d'un acide ou une base de Lewis.

13. Procédé selon la revendication 12, dans lequel ledit acide ou ladite base de Lewis est choisi parmi la pyridine, la 2,6-diterbutylpyridine ; la 2,4,6-triterbutylpyridine ; la 4-Me-2,6-diterbutylpyridine ; Na₂CO₃.

14. Procédé selon la revendication 12 ou 13, dans lequel ledit acide ou ladite base de Lewis est la pyridine.

15. Procédé selon l'une quelconque des revendications 9 à 14, dans lequel l'étape (ii) est réalisée selon une réaction de Heck.

16. Procédé selon la revendication 15, dans lequel ladite réaction est conduite avec un composé de formule (V) : 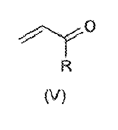 dans laquelle R est défini comme dans la formule (I).

17. Procédé selon l'une quelconque des revendications 9 à 16, dans lequel l'étape (ii) est réalisée *in situ,* tout de suite après l'étape (i), ou après isolement du composé (IV), ou sur le produit brut de l'étape (i) après évaporation du solvant.

18. Procédé selon l'une quelconque des revendications 15 à 17, dans lequel l'étape (ii) est réalisée en présence d'une base.

19. Procédé selon l'une quelconque des revendications 15 à 18, dans lequel l'étape (ii) est réalisée en présence d'un catalyseur au palladium.

20. Procédé selon la revendication 19, dans lequel ledit catalyseur est Pd(PPh₃)Cl₂.

21. Procédé selon l'une quelconque des revendications 9 à 20, comprenant en outre l'étape consistant à isoler le composé de formule (I) obtenu.

22. Procédé de préparation d'un composé de formule (II): 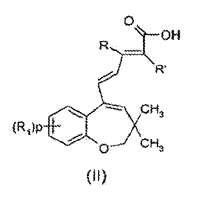 dans laquelle R, R₁, p sont définis comme dans l'une quelconque des revendications 1 à 7 et R' représente un atome d'hydrogène ou un alkyle (en C₁ à C₅) ou un aryle (en C₆ à C₁₀), chacun étant facultativement substitué par un atome d'halogène, par un groupe alkyle (en C₁ à C₅) facultativement halogéné ou par un groupe alcoxy (en C₁ à C₅) facultativement halogéné comprenant les étapes consistant à:
a) réaliser une réaction de Wittig ou un Horner-Emmons/Wadsworth-Emmons sur un composé de formule (I), comme défini dans l'une quelconque des revendications 1 à 8,
b) hydrolyser le produit résultant.

23. Procédé selon la revendication 22, dans lequel R' représente un atome d'hydrogène.

24. Procédé selon la revendication 22 ou 23, dans lequel l'étape a) est réalisée avec un composé de formule b 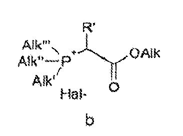 dans laquelle Alk, Alk', Alk", Alk"' représentent indépendamment un groupe alkyle (en C₁ à C₅) et R' est défini comme dans la revendication 22, ou de formule a : 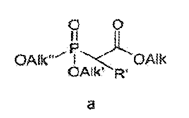 dans laquelle Alk, Alk', Alk" représentent indépendamment un groupe alkyle (en C₁ à C₅) et R' est défini comme dans la revendication 22.

25. Procédé selon l'une quelconque des revendications 22 à 24, dans lequel une base est mise à réagir avec un composé (a) ou avec un composé (b).

26. Procédé selon la revendication 25, dans lequel ladite hase est choisie parmi les hydrures de métal alcalin et les alcoxydes de métal alcalin.

27. Procédé selon l'une quelconque des revendications 21 à 26, dans lequel l'étape b) comprend l'étape consistant à hydrolyser le produit obtenu en le composé acide souhaité de formule (II).

28. Procédé selon la revendication 27, dans lequel ladite hydrolyse est conduite dans des conditions acides.

29. Procédé selon l'une quelconque des revendications 22 à 28, qui comprend en outre l'étape consistant à isoler le composé de formule (II) obtenu.
